# FASCICULE DE BREVET EUROPEEN

(11) **EP 0 568 618 B1**
(45) Date de publication et mention de la délivrance du brevet: **08.10.1997**
(21) Numéro de dépôt: 92904699.3
(22) Date de dépôt: 22.01.1992
(51) Int. Cl.: C08B 37/00, A61K 7/48

(54) **PRODUIT COMPLEXE DE POLYOSE ET D'ACIDE GRAS A FORTE TENEUR EN ACIDE GRAS, UTILISATION COMME AGENT EMULSIONNANT OU HYDRATANT ET COMPOSITION EMULSIONNANTE OU HYDRATANTE EN CONTENANT**
KOMPLEXE VERBINDUNG AUS POLYOSE UND EINER FETTSÄURE MIT EINEM HOHEN GEHALT AN FETTSÄURE, VERWENDUNG ALS EMULGATOR UND FEUCHTHALTENDES MITTEL UND ZUSAMMENSETZUNG WELCHE DIESE VERBINDUNGEN ENTHALTEN
POLYOSE AND FATTY ACID COMPLEX WITH HIGH FATTY ACID CONTENT, USE AS EMULSIFYING OR MOISTURIZING AGENT, AND EMULSIFYING OR MOISTURIZING COMPOSITION CONTAINING IT

(30) Priorité: 23.01.1991 FR 9100758
(43) Date de publication de la demande: 10.11.1993
(73) Titulaire: COLETICA, 69007 Lyon (FR)
(72) Inventeur: HUC, Alain, F-69110 Ste-Foy-Lès-Lyon (FR); ANTONI, Danièle 23, domaine des Essarts, F-69390 Vernaison (FR); PERRIER, Eric, F-38200 Vienne (FR)
(74) Mandataire: Portal, Gérard
(86) Numéro de dépôt international: FR9200054
(87) Numéro de publication internationale: WO9213006

(56) Documents cités:
- EP-A- 0 463 245
- FR-A- 2 009 161
- US-A- 2 749 276
- Ullmann's Encyclopedia of Industrial Chemistry, 5th edn., vol. B2, VCH Weinheim/DE, 1988, page 4-29
- Remington's Pharmaceutical Sciences, 15th edn., 1975, Mack Publishing Co., Easton, Pennsylvania/US, pages 1361,1483

## Description

La présente invention concerne essentiellement un produit complexe polyose-acide gras à forte teneur en acide gras, son utilisation comme agent émulsionnant ou hydratant, ainsi qu'une composition émulsionnante ou hydratante en contenant et notamment une composition cosmétique et/ou pharmaceutique.

On sait que l'hydratation de la peau reste un des grands objectifs de la cosmétique. Pour cela, divers ingrédients actifs sont utilisés. Ils appartiennent à deux grandes catégories : dans la première catégorie, se trouvent des composés à faible poids moléculaire et dans la seconde catégorie, se trouvent des substances de haut poids moléculaire. La première famille peut apporter des hydratations fortes sur un temps relativement court alors que dans le cas de la deuxième famille, l'effet est moins important mais il dure plus longtemps. Parmi les substances hydratantes de faible poids moléculaire, on peut citer par exemple le glycérol, l'urée, les constituants du facteur d'hydratation naturelle ("natural moisturizing factor" ou N.M.F). En ce qui concerne les substances hydratantes de masse moléculaire élevée, en général de type polymérique, elles comprennent en particulier certaines protéines telles que le collagène.

On sait par ailleurs que le ciment intercellulaire de la couche cornée est constitué par un ensemble complexe hydrophobe de lipides constitués essentiellement de stérols libres, d'acides gras libres, de triglycérides, de glycosphingolipides et de céramides. Les molécules hydrophiles utilisées comme hydratant vont être repoussées par ce milieu hydrophobe et elles vont avoir à se diriger vers les couches profondes de l'épiderme et surtout vers le derme qui contient une grande quantité d'eau. De ce fait, la couche cornée et les couches supérieures de l'épiderme subiront très peu l'influence des substances hydratantes. Or, l'impression de sécheresse de la peau provient du Stratum Cornéum et des couches supérieures de l'épiderme. Il s'avère donc important de pouvoir maintenir le plus longtemps possible dans ces couches les molécules hydratantes utilisées.

Il a été décrit dans le document JP-B-46-9327 un procédé de fabrication d'ester d'acide gras de chondroïtine sulfate par réaction d'un halogénure d'acide gras avec les groupes hydroxy libres dans la partie saccharidique du chondroïtine sulfate. Cependant, la proportion d'acide gras dans le produit de réaction obtenu est faible. Ce produit de réaction ne présente pas de propriétés hydratantes significatives et encore moins une activité émulsionnante. D'autres documents similaires sont constitués par les documents JP-A-6-229380 (publication No. JP-A-68/17 566 ; JP-A-02/169684 ; JP-A-62/238209 ; JP-A-02/243611. Le document US-A-4 223 023 décrit un produit liant de lipides non absorbables à base de chitosane, en particulier sous forme de complexes d'acide gras de chitosane par neutralisation du chitosane avec des acides gras (colonne 1, lignes 42 à 48). Ce produit est appliqué comme liant de lipide et en particulier comme produit étendeur de graisse dans les margarines, les sauces, notamment de salades (page 2, lignes 37 à 41). Ce produit diminue également les capacités calorifiques de l'alimentation (page 2, lignes 33 à 36).

Les documents Novak US 2,749,276 et Gillette FR-A-2009161 décrivent des esters d'acides gras et de polyoses préparés en milieu organique à température élevée qui ne sont pas hydrosolubles mais solubles dans des solvants organiques. Ces esters n'ont pas de propriété émulsionnante ou hydratante.

La présente invention a pour but principal de résoudre le nouveau problème technique consistant en la fourniture d'une solution permettant de trouver de nouveaux produits complexes à base de polyose ayant une forte proportion en acide gras, cette proportion étant au moins égale à 10 % en poids encore de préférence au moins égale à 15 % en poids en acide gras par rapport au polyose.

La présente invention a encore pour but principal de résoudre le nouveau problème technique consistant en la fourniture d'une solution permettant de trouver de nouvelles substances ayant un fort pouvoir hydratant et capables, lorsqu'elles sont appliquées sur l'épiderme, de rester dans le Stratum Corneum et dans les couches supérieures de l'épiderme, afin d'améliorer l'effet hydratant.

Pour atteindre ce but, les inventeurs ont eu l'idée de créer des complexes hydrolipidiques constitués de polyoses liés à des acides gras, en particulier par voie chimique. Il s'avère que les polyoses présentent un puissant pouvoir hydratant.

Après obtention de ces complexes hydrolipidiques polyoses-acides gras, il a été découvert que ces nouveaux complexes présentent de façon inattendue des propriétés émulsionnantes et que, de ce fait, ils permettent de réaliser des émulsions sans adjonction d'un autre agent émulsionnant.

Ainsi, la présente invention a encore pour but de résoudre le nouveau problème technique consistant en la fourniture d'une solution permettant de fournir de nouveaux agents émulsionnants biocompatibles permettant d'éviter l'utilisation d'agents de surface synthétiques qui présentent toujours des toxicités plus ou moins importantes.

La présente invention a encore pour but de fournir de nouvelles compositions hydratantes ou émulsionnantes présentant un excellent pouvoir hydratant ou émulsionnant et une toxicité moindre permettant un emploi fréquent dans le domaine cosmétique ou pharmaceutique.

Enfin, la présente invention a encore pour but de fournir de nouvelles compositions cosmétiques ou pharmaceutiques présentant un fort pouvoir hydratant, ne contenant pas d'agent de surface synthétique. Avantageusement, ces compositions cosmétiques ou pharmaceutiques se présenteront sous forme d'émulsion, sans emploi d'agent de surface synthétique.

Tous ces buts sont atteints simultanément selon la présente invention d'une manière simple, peu coûteuse et donc utilisable à l'échelle industrielle.

Ainsi, selon un premier aspect, la présente invention fournit un produit dit complexe à base de polyose, caractérisé en ce qu'il est formé par le milieu réactionnel résultant de la réaction d'au moins un polyose mis en suspension ou dissous dans l'eau, avec au moins un acide gras sous forme réactive, ce milieu réactionnel comprenant une proportion relative en acide gras d'au moins 10 % en poids par rapport au polyose, et en ce que le milieu réactionnel, après avoir été amené à une valeur de pH égale à celle du pH physiologique de l'épiderme, a subi une lyophilisation en se présentant ainsi sous forme d'un produit complexe lyophilisé.

Selon un mode de réalisation particulier, le pH du milieu réactionnel est amené à une valeur supérieure à 7 et au plus égale à 8.

Selon un autre mode de réalisation particulier, l'acide gras sous forme réactive est sous forme d'halogénure d'acide gras, tel que chlorure, fluorure ou à la rigueur iodure, ou sous forme d'anhydride d'acide gras. Un chlorure d'acide gras ou l'anhydride constitue la forme préférée de l'acide gras initial mis à réagir avec le polyose.

Avantageusement, le polyose précité est choisi parmi le groupe consistant de mucopolysaccharides ou glycosaminoglycannes en particulier les glycosamines de structure choisis parmi le groupe consistant de l'acide hyaluronique, chondroïtine 4-sulfate, chrondroïtine 6-sulfate, dermatane sulfate, héparane sulfate et kératane sulfate ou les glycosaminoglycannes de sécrétion, ainsi que l'héparine et ses dérivés ou ses fractions de plus faible masse moléculaire encore dénommées héparine bas poids moléculaire, en abrégé HBPM. Des héparines bas poids moléculaire, compris généralement entre 2 000 et 10 000 sont par exemple décrites dans le brevet CHOAY US-A-4,486,420. D'autres polyoses sont le dextran, le dextrane, des amyloses, des amylopectines et le chitosane.

Selon une variante de réalisation particulière, il s'agit d'un produit complexe obtenu à partir d'un extrait brut de plante terrestre ou aquatique, en particulier marine, telle qu'une algue, contenant une proportion relativement faible en au moins un polyose et qui est mis à réagir avec au mois un acide gras. De tels extraits bruts sont disponibles dans le commerce. On préfère utiliser un extrait de plante marine, en particulier un extrait brut d'algues ascophyllum ou d'algues laminaires.

Selon une autre variante de réalisation particulière, l'acide gras précité est constitué par tout acide gras qui est capable d'être fixé un polyose. Il n'est pas connu actuellement d'exemple d'acide gras qui ne pourrait pas se fixer aux polyoses. Cette fixation peut être réalisée par utilisation d'acide gras initial sous forme d'halogénure ou d'anhydride. Comme acides gras, on peut citer tout acide gras saturé ou insaturé (mono- ou poly-insaturé), en particulier ceux ayant de 8 à 28 atomes de carbone. Des exemples particulièrement avantageux d'acides gras sont l'acide stéarique, l'acide laurique, l'acide linoléique, l'acide linolénique, l'acide undécylénique.

Selon une variante de réalisation avantageuse, on peut utiliser le polyose sous forme d'un mélange polyose-protéine. Un exemple préféré de protéines est l'atélocollagène.
Un produit complexe de l'invention particulièrement préféré est le complexe chondroïtine-4-sulfate-stéarate ainsi que le complexe chitosane-stéarate.

Les proportions relatives d'acide gras par rapport au polyose peuvent varier dans de larges limites, par exemple jusqu'à 75 % en poids. Une proportion relative préférée d'acide gras par rapport au poids du polyose est comprise entre au moins 10 et 75% en poids et de préférence entre 15 et 75% en poids. Ainsi, la proportion relative en acide gras est très élevée par rapport au polyose, cette proportion ne pouvant pas être obtenue simplement par réaction chimique du polyose avec l'acide gras sous forme réactive. Une telle proportion aussi élevée est obtenue de manière critique par l'emploi d'une étape de lyophilisation. Avant de réaliser cette étape, il est également préféré d'amener le pH du milieu réactionnel à une valeur basique, donc supérieure à 7, pour compléter la réaction chimique de couplage. Compte tenu du fait que, dans le cas de l'invention, on préfère préparer des produits hydratants ou émulsionnants, destinés à être utilisés pour la fabrication de compositions cosmétiques ou pharmaceutiques, on préfère amener le pH au voisinage de 8 pour être proche du pH physiologique de l'épiderme.

On a observé que, pour obtenir une activité hydratante et significative du produit de réaction polyose-acide gras, la proportion relative en acide gras est avantageusement comprise entre 10 et 75 % en poids et de préférence entre 15 et 75 % en poids. D'autre part, pour obtenir une activité émulsionnante significative du produit de réaction polyose-acide gras, il est préféré un rapport relatif acide gras/polyose compris entre 50 % et 75 % en poids.

Par ailleurs, selon une caractéristique avantageuse de l'invention, la proportion relative de protéines, lorsqu'elle est présente, peut varier dans de larges limites et, par exemple, sans limitation aller de 0 à 75 % en poids par rapport au poids du polyose. Une source de protéine particulièrement intéressante est formée par un extrait classique disponible dans le commerce de plantes terrestres ou marines, telles que des algues. En effet, de tels extraits contiennent à l'état brut essentiellement un mélange protéine-polyose directement utilisable pour former le produit complexe de l'invention. Ces extraits constituent également une source pratique de polyoses.

La présente invention concerne, selon un deuxième aspect, l'utilisation du produit complexe polyose-acide gras précité comme agent émulsionnant. Dans cette utilisation, toute proportion du produit complexe précité peut être utilisée. Des proportions en poids particulièrement préférées pour lesquelles on obtient un effet émulsionnant varient entre 0,1 et 5 %. Des proportions préférées sont de l'ordre de 1 à 3% en poids par rapport au poids total de la composition à émulsionner.

Selon un troisième aspect, la présente invention concerne également l'utilisation du produit complexe polyose-acide gras précité comme agent hydratant. Ce produit complexe peut être utilisé en toute proportion permettant d'obtenir cet effet hydratant. Des proportions particulièrement préférées varient également entre 0,1 et 5 % en poids par rapport au poids total de la composition à hydrater.

Selon un quatrième aspect, la présente invention concerne également une composition hydratante, caractérisée en ce qu'elle comprend un produit complexe polyose-acide gras tel que précédemment défini.

Selon un cinquième aspect, la présente invention fournit également une composition émulsionnante, caractérisée en ce qu'elle contient à titre d'agent émulsionnant un produit complexe polyose-acide gras tel que précédemment défini.

Selon un sixième aspect, la présente invention concerne encore une composition cosmétique ou pharmaceutique, en particulier à usage topique, caractérisée en ce qu'elle contient un produit complexe polyose-acide gras tel que précédemment défini.

Selon encore un autre aspect, la présente invention concerne encore un procédé de préparation du produit complexe polyose-acide gras précité, caractérisé en ce qu'on fait réagir chimiquement au moins un polyose en suspension ou dissous dans l'eau avec au moins un acide gras sous sa forme réactive d'halogénure ou d'anhydride, on amène le milieu réactionnel à une valeur de pH égale à celle du pH physiologique de l'épiderme, puis on soumet le milieu réactionnel à une étape de lyophilisation, en obtenant ainsi un produit de réaction polyose-acide gras à teneur élevée en acide gras.

Selon un mode de réalisation particulièrement avantageux, une fois la réaction chimique de couplage réalisée, on amène le pH du milieu réactionnel à une valeur de pH basique, ce qui permet de compléter la réaction chimique de couplage des acides gras au polyose, avant de procéder à l'étape de lyophilisation.

Dans le cas particulier où le produit complexe polyose-acide gras est destiné à être utilisé pour la fabrication de compositions cosmétiques ou pharmaceutiques appliquées sur un mammifère, en particulier un être humain, le pH du milieu réactionnel est de préférence amené à une valeur environ égale à 8 pour être proche du pH physiologique.

Il est à noter que, selon une variante de réalisation préférée, l'étape de lyophilisation n'est réalisée que lorsque la valeur de pH a été stabilisée à la valeur basique souhaitée, ce qui peut nécessiter une période de temps relativement longue qui peut être de plusieurs heures.

L'invention concerne encore un procédé de traitement cosmétique ou esthétique de l'épiderme, notamment d'un épiderme ayant un déséquilibre d'hydratation, caractérisé en ce qu'on applique une quantité efficace pour restaurer l'hydratation de l'épiderme d'un produit complexe de polyose-acide gras tel que précédemment défini. Le produit de l'invention améliore ou restaure la plasticité de l'épiderme.

Comme il a été dit précédemment, le produit complexe polyose-acide gras selon la présente invention présente un puissant pouvoir hydratant et de façon inattendue un fort pouvoir émulsionnant sans adjonction d'un autre agent émulsionnant. Ainsi, ce produit complexe permet d'éviter l'emploi d'agent de surface synthétique qui présente toujours des toxicités plus ou moins importantes. Grâce au pouvoir émulsionnant, les compositions contenant le produit complexe de l'invention sont plus naturelles et présentent un toucher cosmétique extrêmement agréable. D'autre part, dans le cas où le polyose utilisé a une action sur le kératinocyte, il est également observé de manière inattendue que le produit complexe selon l'invention permet de renforcer cette propriété au niveau de l'épiderme. Egalement, étant donné que Les glycosaminoglycannes sont des substances constitutives des tissus conjonctifs, le produit complexe selon l'invention présente un grand intérêt en cosmétologie en application pharmaceutique. Ce produit complexe permet, en plus du puissant pouvoir hydratant, de jouer un rôle dans le développement cellulaire.

D'autres buts, caractéristiques et avantages de l'invention apparaîtront clairement à la lumière de la description explicative qui va suivre faite en référence à plusieurs exemples de l'invention donnés simplement à titre d'illustration et qui ne sauraient donc en aucune façon limiter la portée de l'invention. Tous les pourcentages sont donnés en poids, sauf indication contraire.

### Exemple 1

### Préparation du produit complexe (chondroïtine-4-sulfate-acide stéarique)

### A) - Préparation de chondroïtine-4-sulfate

Cet acide est extrait des cloisons nasales de veau. Celles-ci sont tout d'abord lavées soigneusement dans une solution de chlorure de sodium à 9 pour 1 000. Elles sont ensuite hachées et broyées. Le broyat est alors placé à raison de 1 kg/l dans une solution de potasse à 0,5 N. Après agitation, l'ensemble est laissé au repos à température ambiante pendant 24 h. Au bout de ce laps de centrifugation à 30 000 g pendant 50 min, de l'acide acétique pur est ajouté au surnageant pour neutraliser la soude. La solution est ensuite concentrée cinq fois par évaporation sous vide. Le concentrat est versé dans trois fois son volume d'éthanol. Le précipité, récupéré par décantation, est dissous dans l'eau permutée. L'acide chondroïtine-4-sulfate est obtenu par lyophilisation de cette solution.

### B) - Couplage

1 kg de chondroïtine 4-sulfate obtenu selon A) ci-dessus est introduit dans 10 l d'eau. La dissolution est assurée par une agitation de 1 h à l'aide d'une turbine centripète. Le pH de la solution est amené à 8,5 par une solution de soude N.

3 kg de chlorure d'acide stéarique sont alors ajoutés à la solution de chondroïtine-4-sulfate. L'ensemble est agité par la turbine pendant 2 h. Lorsque le pH est inférieur ou égal à 1, celui-ci est porté sous agitation à 8 à l'aide de la lessive de soude. L'ajustement est réalisé par une solution de soude N. Plusieurs heures sont nécessaires pour stabiliser le pH à la valeur 8.

Lorsque le pH est stable, le mélange est lyophilisé dans les conditions suivantes :
* Congélation :
   - Température : -40°C,
   - Durée : 3 h.
* Sublimation :
   - Température : 25°C,
   - Pression : 400 µbar,
   - Durée : 23 h.
* Dessiccation à sec :
   - Température : 40°C,
   - Pression : 5 µbar,
   - Durée : 3 h.

Le lyophilisat obtenu peut être utilisé pour la réalisation d'émulsion.

La composition du produit complexe obtenue, sous forme lyophilisée, est environ la suivante :
- Chondroïtine-4-sulfate : 22 %,
- Acide gras : 65 % (en stéarate de sodium),
- Chlorure de sodium : 13 %.

### Exemple 2

### Préparation portant sur un produit complexe de l'invention chitosane-acide stéarique

### A) - Préparation du chitosane

Le chitosane est un produit disponible dans le commerce qui provient de la désacétylation de la chitine, polymère constituant la carapace des crustacés.

### B) - Couplage

1 kg de chitosane est introduit dans 10 l d'eau dont le pH est amené à 3 par une solution d'acide chlorhydrique N/10. La dissolution est obtenue par une agitation de 1 h à l'aide d'une turbine centripète. Le pH de la solution est alors amené à 8,5 par une solution de soude N.

2,3 kg de chlorure d'acide stéarique sont alors ajoutés à la solution de chitosane. L'ensemble est agité par la turbine pendant 2 h. Lorsque le pH est inférieur ou égal à 1, après les 2 h de réaction, celui-ci est porté à 8 sous agitation à l'aide de la lessive de soude. L'ajustement est réalisé par une solution de soude N. Plusieurs heures sont nécessaires pour stabiliser le pH à la valeur 8.

Lorsque le pH est stable, le mélange est alors lyophilisé dans les conditions de lyophilisation décrites à l'exemple 1.

Le lyophilisat obtenu peut être utilisé pour la réalisation d'émulsions.

### Exemple 3

### Composition cosmétique ou pharmaceutique en émulsion

La formule de cette préparation réalisée à partir du produit complexe de l'exemple 1 est donnée dans le tableau I. Son mode d'obtention est décrit ci-après :

La phase A aqueuse est chauffée à 80°C sous agitation jusqu'à dissolution totale de la poudre du produit complexe de l'invention obtenue à l'exemple 1. Cette opération dure environ 1/2 h.

En même temps, la phase grasse B est chauffée à 80°C sous agitation pendant 20 min de façon à obtenir une homogénéisation complète.

A 80°C, la phase B est ajoutée à la phase A et l'ensemble est homogénéisé par agitation ultrasonique pendant 1 min puis agité lentement à l'aide d'un agitateur centripète. L'agitation est maintenue jusqu'à refroidissement du mélange qui devient de plus en plus visqueux. Lorsque la température atteint 30°C, la partie C est ajoutée à l'émulsion.

### Exemple 4

### Composition cosmétique ou pharmaceutique en émulsion

La formule de cette préparation réalisée à partir du produit complexe de l'exemple 2 est donnée dans le tableau II :

Son mode d'obtention est le suivant :

La phase aqueuse A est chauffée à 80°C sous agitation jusqu'à dissolution totale de la poudre du produit complexe obtenue à l'exemple 2. Cette opération dure environ 30 min.

En même temps, la phase grasse B est chauffée à 80°C sous agitation pendant 20 min de façon à obtenir une homogénéisation complète.

A 80°C, la phase B est ajoutée à la phase A et l'ensemble est homogénéisé par agitation ultrasonique pendant 1 min, puis agité lentement à l'aide d'un agitateur centripète. L'agitation est maintenue jusqu'à refroidissement du mélange qui devient de plus en plus visqueux. Lorsque la température atteint 30°C, la partie C est ajoutée à l'émulsion.

Bien entendu, cette technique s'applique à l'ensemble des polyoses ou aux mélanges polyoses-protéines. De ce fait, des complexes utilisant comme source de polyose des extraits de plantes ou d'algues disponibles dans le commerce peuvent être préparés.

Enfin, il est possible d'utiliser un grand nombre d'acides gras en particulier, des acides gras polyinsaturés, qui peuvent être greffés par l'intermédiaire :
- soit de leur chlorure,
- soit de leur anhydride.

**Tableau I**

| Composés | | Pourcentage en poids |
|---|---|---|
| Phase A | Produit complexe de l'exemple 1 lyophilisé | 3 |
| | Carbopol® | 0 |
| | Eau | 77,7 |
| | Propylèneglycol | 0 |
| | Glycérine | 5 |
| Phase B | Cire d'abeille | 3 |
| | Lanette 14® (1) | 4 |
| | Dragoxat® (2) | 3 |
| | Pur cellin solide® (2) | 4 |
| Phase C | Silicone | 0,5 |
| | Phenonip® (3) | 0,5 |
| | Bactéricide MB® (2) | 0,5 |
| | Triéthanolamine | 0 |

| | | |
|---|---|---|
| 1 - Sté HENKEL | | |
| 2 - Sté DRAGOCO | | |
| 3 - NIPA LABORATORIES | | |

**Tableau II**

| Composés | | Pourcentage en poids |
|---|---|---|
| Phase A | Produit complexe de l'exemple 2 lyophilisé | 1 |
| | Carbopol® | 0,5 |
| | Eau | 79,3 |
| | Propylèneglycol | 2 |
| | Glycérine | 0 |
| Phase B | Cire d'abeille | 3 |
| | Lanette 14® (1) | 3 |
| | Dragoxat® (2) | 3 |
| | Pur cellin solide® (2) | 3 |
| Phase C | Silicone | 0,5 |
| | Phenonip® (3) | 0,5 |
| | Bactéricide MB® (2) | 0,5 |
| | Triéthanolamine | 1 |

| | | |
|---|---|---|
| 1 - Sté HENKEL | | |
| 2 - DRAGOCO | | |
| 3 - NIPA LABORATORIES | | |

## Revendications

1. Produit complexe à base de polyose, caractérisé en ce qu'il est formé par le milieu réactionnel résultant de la réaction d'au moins un polyose mis en suspension ou dissous dans l'eau, avec au moins un acide gras sous forme réactive, ce milieu réactionnel comprenant une proportion relative en acide gras d'au moins 10 % en poids par rapport au polyose, et en ce que le milieu réactionnel, après avoir été amené à une valeur de pH égale à celle du pH physiologique de l'épiderme, a subi une lyophilisation en se présentant ainsi sous forme d'un produit complexe lyophilisé.

2. Produit selon la revendication 1, caractérisé en ce que le pH du milieu réactionnel est amené à une valeur supérieure à 7 et au plus égale à 8.

3. Produit selon l'une des revendications 1 à 2, caractérisé en ce que l'acide gras sous forme réactive est sous forme d'halogénure ou d'anhydride.

4. Produit selon l'une des revendications 1 à 3, caractérisé en ce que le polyose est choisi parmi le groupe consistant d'un glycosaminoglycanne de structure ou de sécrétion ou le chitosane, le dextran, le dextrane, des amyloses, des amylopectines.

5. Produit selon l'une des revendications 1 à 4, caractérisé en ce que l'acide gras précité est un acide gras saturé ou insaturé ayant de 8 à 28 atomes de carbone.

6. Produit selon la revendication 5, caractérisé en ce que l'acide gras est choisi parmi l'acide stéarique, l'acide laurique, l'acide linoléique, l'acide linolénique, l'acide undécylénique.

7. Produit selon l'une des revendications 1 à 6, caractérisé en ce que le polyose est sous forme d'un mélange polyose-protéines dans lequel la proportion de protéine peut aller jusqu'à 75% en poids par rapport au poids du polyose.

8. Produit selon l'une des revendications 4 à 7, caractérisé en ce que le glycosaminoglycanne de structure ou de sécrétion est choisi parmi l'acide hyaluronique, le chondroïtine-4-sulfate, le chondroïtine-6-sulfate, le dermatane sulfate, l'héparane sulfate, le kératane sulfate, l'héparine et ses dérivés, les héparines de bas poids moléculaire ou HBPM.

9. Produit selon l'une des revendications 1 à 8, caractérisé en ce qu'il est obtenu à partir d'un extrait de plante terrestre ou marine, une algue, contenant au moins un polyose.

10. Produit selon la revendication 9, caractérisé en ce que l'algue est ascophyllum ou laminaire

11. Produit selon l'une des revendications 1 à 8, caractérisé en ce qu'il comprend un produit de réaction choisi parmi le chondroïtine-4-sulfate-stéarate et le chitosane-stéarate.

12. Produit selon l'une des revendications 1 à 9, caractérisé en ce que la proportion relative acide gras/polyose est comprise entre au moins 10 et 75 % en poids.

13. Produit selon la revendication 11, caractérisé en ce que l'on utilise comme réactif de départ le chondroïtine-4-sulfate et l'acide stéarique sous forme réactive, le milieu réactionnel lyophilisé comprend 22 % en poids de chondroïtine-4-sulfate et 65 % en poids d'acide gras sous forme de stéarate de sodium, où l'on utilise comme réactif de départ 1 kg de chitosane pour 2,3 kg de chlorure d'acide stéarique et le milieu réactionnel est stabilisé à un pH de 8 avant d'être lyophilisé.

14. Utilisation du produit complexe tel que défini dans l'une quelconque des revendications 1 à 13, comme agent émulsionnant.

15. Utilisation du produit complexe tel que défini dans l'une quelconque des revendications 1 à 12, comme agent hydratant.

16. Composition hydratante, caractérisée en ce qu'elle comprend à titre d'agent hydratant un produit complexe polyose-acide gras tel que défini dans l'une quelconque des revendications 1 à 13.

17. Composition émulsionnante, caractérisée en ce qu'elle comprend, à titre d'agents émulsionnants, un produit complexe polyose-acide gras tel que défini dans l'une quelconque des revendications 1 à 13.

18. Composition cosmétique ou pharmaceutique, en particulier à usage topique, caractérisée en ce qu'elle comprend un produit complexe polyose-acide gras tel que défini à l'une quelconque des revendications 1 à 13.

19. Composition selon la revendication 16, 17 ou 18, caractérisée en ce qu'elle comprend ledit produit complexe polyose-acide gras, à une proportion comprise entre 0,1 et 5 % en poids par rapport au poids total de la composition.

20. Procédé de préparation d'un produit complexe polyose-acide gras tel que défini à l'une quelconque des revendications 1 à 13, caractérisé en ce qu'on fait réagir chimiquement au moins un polyose en suspension ou dissous dans l'eau avec au moins un acide gras sous sa forme réactive d'halogénure ou d'anhydride, on amène le milieu réactionel à une valeur de pH égale à celle du pH physiologique de l'épiderme, puis on soumet le produit réactionnel basique à une étape de lyophilisation.

21. Procédé de traitement cosmétique ou esthétique de l'épiderme, caractérisé en ce qu'on applique une quantité efficace pour restaurer l'hydratation de l'épiderme d'un produit complexe de polyose-acide gras tel que défini à l'une quelconque des revendications 1 à 13.

## Patentansprüche

1. Komplexes Produkt auf der Basis von Polyose, dadurch gekennzeichnet, daß es durch ein Reaktionsmedium gebildet wird, das durch das Umsetzen zumindest einer in Wasser suspendierten oder gelösten Polyose mit zumindest einer Fettsäure in reaktiver Form erhalten wird, wobei dieses Reaktionsmedium eine relative Menge an Fettsäure von zumindest 10 %, bezogen auf die Masse der Polyose, umfaßt, und daß das Reaktionsmedium, nachdem es auf einen pH-Wert gleich jenem des physiologischen pH der Epidermis geführt worden war, einer Lyophilisation unterworfen wurde, wobei es so in Form eines lyophilisierten komplexen Produkts vorliegt.

2. Produkt nach Anspruch 1, dadurch gekennzeichnet, daß der pH des Reaktionsmediums auf einen Wert von mehr als 7 und höchstens 8 geführt wird.

3. Produkt nach einem der Ansprüche 1 oder 2, dadurch gekennzeichnet, daß die Fettsäure in reaktiver Form in Form eines Halogenids oder Anhydrids vorliegt.

4. Produkt nach einem der Ansprüche 1 bis 3, dadurch gekennzeichnet, daß die Polyose ausgewählt ist aus der Gruppe bestehend aus einem Struktur- oder Sekretionsglykosaminoglykan oder Chitosan, Dextran, Dextrane, Amylosen, Amylopektinen.

5. Produkt nach einem der Ansprüche 1 bis 4, dadurch gekennzeichnet, daß die Fettsäure eine gesättigte oder ungesättigte Fettsäure mit 8 bis 28 Kohlenstoffatomen ist.

6. Produkt nach Anspruch 5, dadurch gekennzeichnet, daß die Fettsäure ausgewählt ist aus Stearinsäure, Laurinsäure, Linolsäure, Linolensäure, Undecylensäure.

7. Produkt nach einem der Ansprüche 1 bis 6, dadurch gekennzeichnet, daß die Polyose in Form einer Mischung von Polyose-Proteinen vorliegt, worin die Menge an Protein bis zu 75 Masse-%, bezogen auf die Masse der Polyose, betragen kann.

8. Produkt nach einem der Ansprüche 4 bis 7, dadurch gekennzeichnet, daß das Struktur- oder Sekretionsglykosaminoglykan ausgewählt ist aus Hyaluronsäure, Chondroitin-4-sulfat, Chondroitin-6-sulfat, Dermatansulfat, Heparansulfat, Keratansulfat, Heparin und seinen Derivaten, Heparinen mit niedriger Molmasse oder HBPM.

9. Produkt nach einem der Ansprüche 1 bis 8, dadurch gekennzeichnet, daß es aus einem Extrakt einer Land- oder Meerespflanze, einer Alge, die zumindest eine Polyose enthält, erhalten wird.

10. Produkt nach Anspruch 9, dadurch gekennzeichnet, daß die Alge ascophyllisch oder laminar ist.

11. Produkt nach einem der Ansprüche 1 bis 8, dadurch gekennzeichnet, daß es ein Reaktionsprodukt, ausgewählt aus Chondroitin-4-sulfat-Stearat oder Chitosan-Stearat, umfaßt.

12. Produkt nach einem der Ansprüche 1 bis 9, dadurch gekennzeichnet, daß die relative Menge an Fettsäure/Polyose zwischen zumindest 10 und 75 Masse-% liegt.

13. Produkt nach Anspruch 11, dadurch gekennzeichnet, daß als Ausgangsreagenz Chondroitin-4-sulfat und Stearinsäure in reaktiver Form verwendet werden, das lyophilisierte Reaktionsmedium 22 Masse-% Chondroitin-4-sulfat und 65 Masse-% Fettsäure in Form von Natriumstearat umfaßt, oder als Ausgangsreagenz 1 kg Chitosan für 2,3 kg Stearinsäurechlorid verwendet wird, und das Reaktionsmedium auf einem pH von 8 stabilisiert wird, bevor es lyophilisiert wird.

14. Verwendung eines komplexen Produkts, wie in einem der Ansprüche 1 bis 13 definiert, als Emulgator.

15. Verwendung eines komplexen Produkts, wie in einem der Ansprüche 1 bis 12 definiert, als Hydratisierungsmittel.

16. Hydratisierungszusammensetzung, dadurch gekennzeichnet, daß sie als Hydratisierungsmittel ein komplexes Polyose-Fettsäure-Produkt, wie in einem der Ansprüche 1 bis 13 definiert, umfaßt.

17. Emulgatorzusammensetzung, dadurch gekennzeichnet, daß sie als Emulgatoren ein komplexes Polyose-Fettsäure-Produkt, wie in einem der Ansprüche 1 bis 13 definiert, umfaßt.

18. Kosmetische oder pharmazeutische Zusammensetzung, insbesondere zur topischen Verwendung, dadurch gekennzeichnet, daß sie ein komplexes Polyose-Fettsäure-Produkt, wie in einem der Ansprüche 1 bis 13 definiert, umfaßt.

19. Zusammensetzung nach Anspruch 16, 17 oder 18, dadurch gekennzeichnet, daß sie das komplexe Polyose-Fettsäure-Produkt in einer Menge zwischen 0,1 und 5 Masse-%, bezogen auf die Gesamtmasse der Zusammensetzung, umfaßt.

20. Verfahren zur Herstellung eines komplexen Polyose-Fettsäure-Produkts, wie in einem der Ansprüche 1 bis 13 definiert, dadurch gekennzeichnet, daß zumindest eine in Wasser suspendierte oder gelöste Polyose mit zumindest einer Fettsäure in ihrer reaktiven Form eines Halogenids oder Anhydrids chemisch umgesetzt wird, das Reaktionsmedium auf einen pH-Wert gleich jenem des physiologischen pH der Epidermis geführt wird, und dann das basische Reaktionsprodukt einem Lyophilisationsschritt unterworfen wird.

21. Verfahren zur kosmetischen oder ästhetischen Behandlung der Epidermis, dadurch gekennzeichnet, daß eine wirksame Menge eines komplexen Polyose-Fettsäure-Produkts, wie in einem der Ansprüche 1 bis 13 definiert, zur Wiederherstellung der Hydratisierung der Epidermis aufgebracht wird.

## Claims

1. A polyose-based complex product, characterized in that it is formed by the reaction medium resulting from the reaction of at least one polyose that is suspended or dissolved in water, with at least one fatty acid under a reactive form, said reaction medium comprising a relative proportion of fatty acid of at least 10% by weight with respect to the polyose, and in that the reaction medium, after having been brought to a pH value that is equal to the value of the physiological pH of the epidermis, has undergone lyophilization by thus being under the form of a complex lyophilized product.

2. The product according to claim 1, characterized in that the pH of the reaction medium is brought to a value that is greater than 7 and at the most equal to 8.

3. The product according to one of claims 1 to 2, characterized in that the fatty acid under reactive form is under the form of a halide or an anhydride.

4. The product according to one of claims 1 to 3, characterized in that the polyose is selected from the group consisting of a structural or secretory glycosaminoglycan or chitosan, dextran, dextrane, amyloses and amylopectins.

5. The product according to one of claims 1 to 4, characterized in that the aforesaid fatty acid is a saturated or unsaturated fatty acid having from 8 to 28 carbon atoms.

6. The product according to claim 5, characterized in that the fatty acid is selected from the group consisting of stearic acid, lauric acid, linoleic acid, linolenic acid and undecylenic acid.

7. The product according to one of claims 1 to 6, characterized in that the polyose is under the form of a polyose-proteins mixture in which the protein proportion may reach 75% by weight with respect to the weight of the polyose.

8. The product according to one of claims 4 to 7, characterized in that the structural or secretory glycosaminoglycan is selected from hyaluronic acid, chondroitin-4-sulfate, chondroitin-6-sulfate, dermatan sulfate, heparan sulfate, keratan sulfate, heparin and its derivatives, low-molecular weight heparins and HBPM.

9. The product according to one of claims 1 to 8, characterized in that it is obtained from an extract of a terrestrial or marine plant, an alga, said extract containing at least one polyose.

10. The product according to claim 9, characterized in that the alga is ascophyllum or laminaria.

11. The product according to one of claims 1 to 8, characterized in that it comprises a reaction product selected from chondroitin 4-sulfate/stearate and chitosan/stearate.

12. The product according to one of claims 1 to 9, characterized in that the relative proportion of fatty acid to polyose is between at least 10 and 75% by weight.

13. The product according to claim 11, characterized in that chondroitin 4-sulfate and stearic acid, under a reactive form, are used as starting reactant, the lyophilized reaction medium comprises 22% by weight of chondroitin 4-sulfate and 65% by weight of fatty acid under the form of sodium stearate, where 1 kg of chitosan is used as the starting reactant for 2.3 kg of stearic acid chloride and the reaction medium is stabilized to pH 8 before being lyophilized.

14. Use of the complex product as defined in any one of claims 1 to 13, as emulsifier.

15. Use of the complex product as defined in any one of claims 1 to 12, as moisturizer.

16. A moisturizing composition, characterized in that it comprises as moisturizer, a polyose/fatty acid complex product as defined in any one of claims 1 to 13.

17. An emulsifying composition, characterized in that it comprises as emulsifier a polyose/fatty acid complex product as defined in any one of claims 1 to 13.

18. Cosmetic or pharmaceutical composition, in particular for topic use, characterized in that it comprises a polyose-fatty acid complex product as defined in any one of claims 1 to 13.

19. A composition according to claim 16, 17 or 18, characterized in that it comprises said polyose/fatty acid complex product in a proportion ranging between 0.1 and 5% by weight with respect to the total weight of the composition.

20. A method of preparing a polyose/fatty acid complex product as defined in any one of claims 1 to 13, characterized in that at least one polyose suspended or dissolved in water is chemically reacted with at least one fatty acid under its halide or anhydride form, the reaction medium is brought to a pH value equal to that of the physiological pH of the epidermis, and then the basic reaction product is subjected to a lyophilisation step.

21. A method of cosmetic or esthetic treatment of the epidermis, characterized in that an effective amount is applied so as to restore moisturizing to the epidermis of a polyose/fatty acid complex product as defined in any one of claims 1 to 13.
